# EUROPÄISCHE PATENTSCHRIFT

(11) **EP 1 137 800 B1**
(45) Veröffentlichungstag und Bekanntmachung des Hinweises auf die Patenterteilung: **09.04.2003**
(21) Anmeldenummer: 99950544.9
(22) Anmeldetag: 23.09.1999
(51) Int. Cl.: C12Q 1/68

(54) **VERFAHREN ZUR REINIGUNG BZW. ISOLIERUNG VIRALER NUKLEINSÄUREN**
METHOD FOR PURIFYING OR ISOLATING VIRAL NUCLEIC ACIDS
PROCEDE DE PURIFICATION OU D'ISOLEMENT D'ACIDES NUCLEIQUES VIRAUX

(30) Priorität: 08.12.1998 DE 19856415
(43) Veröffentlichungstag der Anmeldung: 04.10.2001
(73) Patentinhaber: Fenner, Thomas, 22587 Hamburg (DE)
(72) Erfinder: FENNER, Thomas, D-22587 Hamburg (DE); PETERSEN, Heiko, D-20251 Hamburg (DE)
(74) Vertreter: UEXKÜLL & STOLBERG
(86) Internationale Anmeldenummer: EP9907093
(87) Internationale Veröffentlichungsnummer: WO00034510

(56) Entgegenhaltungen:
- PETERSEN H. ET AL.,: "Looking for HBV,HCV and HIV-1 in plasmapool samples by nucleic acid amplification (NAT)" VOX SANGUI, Bd. 74, Nr. s1, - Juni 1998 (1998-06) Seite 1099 XP000884505
- "Qiagen product guide 1997" , QIAGEN GMBH, HILDEN XP002132073 Seite 66 -Seite 69
- LIN H.J. ET AL.,: "Improved methods for quantification of human immunodeficiency virus type 1 RNA and hepatitis C virus RNA in blood using spin column technology and chemiluminescent assays of PCR products" J. MED. VIROLOGY, Bd. 51, - 1997 Seite 56-63 XP000877164
- WOLFF C. ET AL.,: "single tube nested PCR with room temperature stable reagents" PCR METHODS AND APPLICATIONS, Bd. 4, - April 1995 (1995-04) Seiten 376-379, XP002132071
- MORNADI P.-A. ET AL.,: "Detection of human immunodeficiency virus type 1 (HIV-1) RNA in pools of sera negative fo anibodies to HIV-1 and HIV-2" J. CLIN. MICROBIOLOGY, Bd. 36, Nr. 6, - Juni 1998 (1998-06) Seiten 1534-1538, XP000869877 in der Anmeldung erwähnt

## Beschreibung

Die vorliegende Erfindung betrifft ein Verfahren zur Isolierung viraler Nukleinsäuren aus Viruspartikel enthaltenden Flüssigkeiten, wie z.B. Plasma oder anderen Flüssigkeiten, wobei die Nukleinsäuren in reiner Form erhalten werden. Insbesondere betrifft die Erfindung den Nachweis von in Flüssigkeiten, vor allem in Plasma-Pools, vorhandenen Viren. Gegenstand der vorliegenden Erfindung ist insbesondere ein Verfahren zur Vorbereitung von Proben für Nukleinsäure-Amplifikationstechniken sowie ein Kit zur Durchführung des Verfahrens.

Die Freigabe von Blutplasma und anderen Blutprodukten, wie z.B. Thrombozyten, zur Applikation beim Menschen hängt in entscheidendem Maße davon ab, ob die Produkte beispielsweise mit Viren kontaminiert sind. Für den hochsensitiven Nachweis und/oder die Quantifizierung verschiedener Viren, wie z.B. Hepatitis B oder C Virus (HBV, HCV) bzw. Humanes Immundefizienzvirus (HIV)-1 oder -2, werden inzwischen häufig auf Nukleinsäure-Amplifikationstechniken (NAT) basierende Assays, wie z.B. auf Polymerase-Kettenreaktion (PCR), branched-DNA-Detection (bDNA) oder Nucleic Acid Sequence Based Amplification (NASBA) basierende Assays, verwendet.

Die Nachweisgrenze der kommerziell erhältlichen Assays, wie z.B. Amplicor HCV®, Amplicor HBV-Monitor® (Hoffmann-La Roche) oder Amplicor HIV-1 Monitor® (Hoffmann-La Roche), liegt bei etwa 100 bis 400 DNA-/RNA-Kopien/ml Plasma. Diese Sensitivität ist für die Untersuchung der Plasmaprobe und den Nachweis der genannten Viren in Plasmaproben von Einzelspendern zwar zufriedenstellend, jedoch ist das Testen von Einzelproben bei steigendem Plasmabedarf aufgrund des hohen Zeit- und Kostenfaktors routinemäßig praktisch nicht durchführbar. Aus diesem Grund ist es zweckmäßiger, Plasma-Pools zu testen, die z.B. aus 96 verschiedenen Einzelproben bestehen.

In diesem Zusammenhang stellt die Nachweisgrenze der kommerziell erhältlichen Assays zum Nachweis von Viren jedoch ein Problem dar. Sollte beispielsweise nur eine von 96 Proben Virus-positiv sein, so würde die Gesamt-Viruskonzentration aufgrund des Verdünnungsfaktors im Plasma-Pool auf etwa 1/100 absinken, d.h. einen Wert, der in der Regel unterhalb der Nachweisgrenze des jeweils verwendeten Assays liegt. Werden zum Beispiel in einer Einzelprobe 1000-4000 RNA-Kopien/ml HCV gemessen, lägen in einem 96er Plasma-Pool (bei Verwendung dieser einen kontaminierten und 95 virusfreien Einzelproben mit jeweils gleichem Volumen) nur etwa 10-40 Kopien/ml HCV vor, die durch die kommerziell erhältlichen Assays bislang nicht nachweisbar sind. Dies hätte die Kontamination des gesamten Plasma-Pools und die Übertragung der Viren auf eine Vielzahl von Plasma-Empfängern zur Folge.

Das für die Überprüfung der Blutbanken verantwortliche Paul-Ehrlich-Institut (PEI) hat ab April 1999 beispielsweise die Freigabe von Erythrozyten- und Thrombozythenkonzentraten über eine Testung auf HCV mittels Nukleinsäure-Amplifikationstechniken (NATs) vorgeschrieben, wobei eine Empfindlichkeit von 5000 IU HCV/ml pro Einzelprobe zu gewährleisten ist (1 IU = 2-6 RNA(Virus)-Kopien).

Dieser Nachweis kann aufgrund der Sensitivität des derzeit erhältlichen NAT-Assays (Amplicor HCV Test) bislang nur an Einzelproben oder kleinen Pools (die bis 48 Proben enthalten) durchgeführt werden. Eine Testung auf HBV und HIV ist dabei nicht möglich.

Bei Blutprodukten, wie z.B. Thrombozyten, die nur für sehr kurze Zeit (3-4 Tage) haltbar sind, stellt der Nachweis kontaminierender Viren ein Problem dar, da der Test mit bislang durchgeführten Anreicherungsverfahren etwa 2 Tage dauert, während kommerziellen Nachweisverfahren, die innerhalb kürzerer Zeiträume durchgeführt werden können, die erforderliche Sensitivität fehlt. Die Verwendbarkeit dieser Produkte nach Abschluß der Viren-Tests ist daher aufgrund der begrenzten Haltbarkeit entweder zeitlich stark eingeschränkt, oder die Applikation ist für den Empfänger (Patienten) mit einem vergleichsweise hohen Infektionsrisiko verbunden.

Im Zusammenhang mit den bislang eingesetzten Nukleinsäure-Amplifikationstechniken ist der Fachmann vor ein weiteres Problem gestellt. Bei der hochsensitiven Isolierung von viralen Nukleinsäuren aus Flüssigkeiten für mehr als einen Parameter (d.h. bei Nachweis von mehr als einem Virustyp pro Probe) ist es generell erforderlich, relativ große Volumina (ca. 5-10 ml) zu bearbeiten. Alle kommerziellen Testsysteme sind jedoch auf den Einsatz von kleinen Volumina (100-200 µl) für nur einen Parameter (d.h. nur einen Virustyp) ausgerichtet.

Um z.B. noch eine Viruskonzentration von ca. 20 Partikeln pro ml Plasma (bei HCV entsprechend ca. 6 IU/ml) nachweisen zu können, sind sie deshalb nur dann geeignet, wenn zuvor eine Konzentrierung der Viren durch Fällung, Ultrazentrifugation oder Filtrierung erfolgt.

Die Präzipitation von Viruspartikeln aus Flüssigkeiten mittels Polyethylenglykol (PEG) ist seit Anfang der 70er Jahre eine Standardtechnik zur Konzentrierung von Viren bei geringer Zentrifugationskraft (Yamamoto et al., Virology 40 (1970) 734; Morandi et al., J. Clin. Microbiol. 36 (1998) 1534-38). In mannigfaltigen Abwandlungen (z.B. Konzentration und Art des verwendeten PEGs) wird hierbei die Flüssigkeit mit PEG und NaCl gemischt. Die Präzipitation erfolgt in der Kälte, und die Virus(Protein)/PEG-Präzipitate werden anschließend durch Zentrifugation gewonnen. Die weiteren Bearbeitungsschritte (Erhalt der vitalen Viruspartikel, Aufschluß der Viruspartikel etc.) hängen schließlich von der wissenschaftlichen Fragestellung ab.

Eine alternative Methode stellt die Ultrazentifugation von Viren in Flüssigkeiten dar. Hierbei wird alleine durch die über längere Zeit einwirkende Zentrifugalkraft eine Anreicherung der Viruspartikel am Boden des Zentrifugationsgefäßes erreicht. Diese Methode ist allerdings für HCV nur eingeschränkt anwendbar, da sich die HCV-Partikel oftmals in der Lipidschicht befinden und somit nicht quantitativ präparierbar sind.

Um an Virus-Nukleinsäuren zu gelangen, bedarf es des Aufschlusses (mechanische oder chemische Desintegration) der Virionen. Dazu findet im Falle von RNA Viren (z.B. HCV oder HIV-1) meistens das chaotrope Denaturans Guanidinisothiocyanat (GIT) Verwendung. GIT denaturiert alle Proteine (auch RNA abbauende RNasen), so daß die Nukleinsäuren frei in Lösung vorliegen und vor Abbau geschützt sind.

Alternativ kann Phenol/Chloroform als denaturierendes Agens eingesetzt werden (Cox, Methods Enzymol. 12B (1968) 120; Glisen et al., Biochemistry 13 (1974) 2633; Ullrich et al., Science 196 (1977) 1313). Dies ist jedoch wegen Toxizität, Entsorgung etc. der verwendeten Agenzien nachteilig.

Die extrahierten Nukleinsäuren können schließlich durch verschiedene Methoden aufgereinigt werden. Neben einer einfachen Alkohol-Fällung aus dem Lösungsgemisch ist die Bindung der Nukleinsäuren an Silicia-Matrices eine weitverbreitete Methode. Kommerziell werden Kits zur Durchführung der Silicia-Methode von den Firmen QIAGEN (Hilden, DE), Macherey-Nagel (Düren, DE), Boehringer-Mannheim (Mannheim, DE), Organon Teknika (Turnhout, BE) und diversen anderen Anbietern vertrieben.

Als Endprodukt erhält man eine in neutraler Lösung (H₂O oder Tris-Puffer) vorliegende, meist hochgereinigte Nukleinsäure (Vogelstein und Gillespie, Proc. Natl. Acad. Sci. USA 76 (1979) Seite 615-619; Boom et al., J. Clin. Microbiol. 28 (1990) 495).

Während die Extraktion von Viruspartikeln aus Ultrazentrifugations-Pellets außer im Falle von HCV (s.o.) im allgemeinen kein Problem darstellt, sind durch PEG-Fällung aufkonzentrierte Viren schlecht zu extrahieren. Nach einer von Morandi et al. beschriebenen Methode wird ein Extraktionspuffer verwendet und nachfolgend mit Alkohol präzipitiert (J. Clin. Microbiol. 36 (1998) 1534-38). Die so gewonnene Nukleinsäure ist allerdings noch mit PEG kontaminiert und eignet sich nur ungenügend für Folgeanwendungen, bei denen hochreine Nukleinsäuren erforderlich sind.

Aufgabe der vorliegenden Erfindung ist es daher, ein Verfahren zur Verfügung zu stellen, mit dem Viren, insbesondere HBV, HCV und HIV, in Blutplasma-Pools nachgewiesen und/oder quantifiziert werden können. Ferner ist es Aufgabe der vorliegenden Erfindung, ein Nachweisverfahren für Viren zur Verfügung zu stellen, das eine hochsensitive und schnelle Testung von Blutprodukten (wie z.B. für Thrombozyten erforderlich) ermöglicht, die nur für relativ kurze Zeit haltbar sind.

Die Aufgabe wird erfindungsgemäß durch ein Verfahren nach den Ansprüchen 1 bis 7 und durch ein Kit nach den Ansprüchen 8 bis 12 gelöst.

Zur Lösung der Aufgabe wird ein Verfahren vorgeschlagen, bei dem man Gesamtnukleinsäuren aus Viruspartikeln, die in Plasma oder anderen Flüssigkeiten vorhanden sind, extrahiert und in reiner Form für nachfolgende Anwendungen, insbesondere NATs, isoliert.

Die vorliegende Erfindung betrifft somit ein Verfahren zur Reinigung bzw. Isolierung von Nukleinsäuren von in flüssigen biologischem Probenmaterial gegebenenfalls vorhanden Viruspartikeln, das dadurch gekennzeichnet ist, daß man
a) eine Probe mit Polyethylenglykol (PEG) und Natriumchlorid versetzt und die Viruspartikel in der Kälte, vorzugsweise bei etwa 4°C, ausfällt,
b) eine Zentrifugation durchführt und
c) erhaltene Pellets in Guanidinisothiocyanat-Puffer (GIT-Puffer: 6 M Guanidinisothiocyanat (GIT), 200 mM Dithiothreitol (DTT), 100 mM Tris, pH 6,5) löst.

Bei dem biologischen Probenmaterial kann es sich um z.B. Plasma-Proben (vorzugsweise aus Plasma-Pools), andere Blutprodukte oder andere Flüssigkeiten handeln, die mit Viren kontaminiert sein können.

Die in Stufe c) erhaltenen RNA- bzw. DNA-Proben können anschließend, nach Aufreinigung, durch NAT auf eine mögliche Kontamination mit Viren untersucht werden. Vorzugsweise kann das Virusangereicherte Gemisch aus Stufe c) unmittelbar in einen kommerziell erhältlichen Kit zur Isolierung viraler DNA/RNA überführt werden.

Gemäß einer besonderen Ausführungsform betrifft die Erfindung ein Verfahren zur Aufreinigung viraler Nukleinsäuren, das die o.g. Schritte a) bis c) sowie die zusätzlichen Stufen d) und e) umfaßt, wobei man
in Stufe d) die in Stufe c) erhaltene Mischung gegebenenfalls mit dem gleichen Volumen Lysis-Puffer eines kommerziell erhältlichen Virus-RNA/DNA-Isolierungskits (z.B. "Buffer AVL" des "QIAmp Viral RNA Kits", QIAGEN oder "Binding Buffer" des "High Pure Viral RNA Kits" Boehringer Mannheim oder anderer Hersteller) mischt, anschließend das doppelte Volumen 96-99 %iges Ethanol zugibt, und man
in Stufe e) eine Reinigung an einer Silicia-Matrix durchführt.

Erfindungsgemäß kann in Stufe d) auf die Zugabe des Lysis-Puffers verzichtet und gleich Ethanol zugegeben werden. Alternativ kann anstelle des Lysis-Puffers auch das gleiche Volumen GIT-Puffer zugegeben werden.

Die gereinigten Nukleinsäuren liegen anschließend im Eluat vor.

Als Lysis-Puffer kommen vorzugsweise alle Puffer in Frage, die vom Hersteller der Virus-DNA/RNA-Isolierungskits mitgeliefert werden, es kommen jedoch auch andere Lysis-Puffer in Frage, die vorzugsweise GIT enthalten.

Zur Reinigung der Virus RNA/DNA sind im Rahmen der vorliegenden Erfindung die üblicherweise mit den Kits mitgelieferten Silicia-Matrices (.B. als Filter, in Form von Perlen etc.) bevorzugt. Es können jedoch auch andere Materialien eingesetzt werden, die RNA bzw. DNA binden und dadurch deren Reinigung ermöglichen.

Das Verfahren dient vorzugsweise zur Vorbereitung von biologischen Proben, insbesondere von Proben aus Plasma-Pools, für den anschließenden Einsatz in NAT, wobei die erhaltenen gereinigten Nukleinsäuren ohne weitere Vorbehandlung direkt in das Amplikationsverfahren eingesetzt werden können. Die vorzugsweise zur Anwendung kommenden NAT (PCR, bDNA, NASBA etc.) sind dem Fachmann wohlbekannt.

Bei den Nukleinsäuren handelt es sich insbesondere um DNA bzw. RNA von HCV, HBV und HIV (insbesondere HIV-1).

Gemäß einer besonderen Ausführungsform der Erfindung wird das Verfahren durchgeführt, indem man
in Stufe a) eine Plasmaprobe verwendet und zu einem Probenvolumen von max. ca. 10 ml etwa 3 Vol.-% PEG 6000 und ca. 150 mM einer wässrigen Natriumchloridlösung zugibt und für ca. 15-20 Min. bei ca. 4°C stehenläßt,
in Stufe b) für 15-20 Min. bei 4°C und ca. 3000 g zentrifugiert,
in Stufe c) zum Lösen des Pellets 300 bis 400 µl GIT-Puffer (s.o.) zugibt und für ca. 10 Min. bei 56°C löst und
in Stufe d) die Lösung mit dem Lysis-Puffer eines kommerziellen Virus-RNA Kits (s.o.) im Verhältnis 1:1 mischt.

Eine besonders bevorzugte Ausführungsform der Erfindung betrifft ein Verfahren, bei dem man
a) 4,8 ml eines 96er Plasmapools in einem 7 ml Zentrifugationsröhrchen mit 300 µl 50%igem PEG 6000 und 150 µl einer 5 M wässrigen Natriumchloridlösung versetzt, mischt und für ca. 15-20 Minuten bei 4°C inkubiert,
b) die Mischung für 15-20 Min. bei 4°C mit 3000 g zentrifugiert,
c) 300 bis 400 µl GIT-Puffer (s.o.) zu dem Pellet gibt und dieses durch Inkubation bei ca. 56 °C für ca. 10 Min. löst,
d) das Lysat mit 300 bis 400 µl GIT-haltigem AVL-Buffer (s.o.) im Verhältnis 1:1 mischt und anschließend mit 600 bis 800 µl 96-99 %igem Ethanol versetzt,
e) eine Reinigung an Silicia-Matrix durchführt,
wobei die gereinigten Nukleinsäuren im Eluat vorliegen.

Bei der Reinigung an Silicia-Matrix (SIO₂, z.B. in Form eines Filters, Perlen etc.) werden die zunächst gebundenen Nukleinsäuren gewaschen und anschließend von der Säule eluiert.

Dieses Verfahren dient vorzugsweise der Probenvorbereitung für den Nachweis von HCV, HBV oder HIV (insbesondere HIV-1) aus gepoolten Plasmen von Blutspendern, wobei man nach Durchführung des erfindungsgemäßen Verfahrens zur Probenvorbereitung die Virus-RNA anschließend mittels bekannter Nukleinsäure-Amplifikationstechniken (z.B. PCR, bDNA oder NASBA) amplifiziert und nach allgemein bekannten Methoden (wie z.B. durch Hybridisierung mit Hilfe markierter Sonden etc.) nachweist.

Die Erfindung betrifft ferner ein Kit zur Durchführung des erfindungsgemäßen Reinigungs- bzw. Isolierungsverfahrens oder zur Durchführung des Verfahrens zur Vorbereitung von biologischen Proben für NATs, der definierte Mengen an PEG, Natriumchlorid, GIT-Puffer und Ethanol in geeigneten Aufbewahrungsgefäßen enthält, die es ermöglichen, z.B. ein vorgegebenes Plasma-Probenvolumen dem Verfahren zu unterziehen.

Vorzugsweise enthält der Kit als Polyethylenglykol PEG 6000 und Natriumchlorid in Form einer wässrigen Lösung, gemäß einer besonderen Ausführungsform 50 % PEG 6000 und 5 M wässrige NaCl-Lösung.

Gemäß einer besonderen Ausführungsform der Erfindung enthält der Kit zur Vorbereitung einer Plasmaprobe von je 4,8 ml Volumen
a) 300 µl 50%-iges PEG 6000,
b) 150 µl einer 5 M wässrigen Natriumchlorid-Lösung,
c) 400 µl GIT-Puffer und
d) 800 µl 96-99 % Ethanol.

Im Rahmen dieser Erfindung wird erstmals ein Kit zum Nachweis von HCV, HBV oder HIV aus gepoolten Plasmen von Blutspendern zur Verfügung gestellt, der zusätzlich zu den zur Durchführung der Nukleinsäure-Amplifikation erforderlichen Bestandteilen die Bestandteile eines der oben genannten Kits zur Durchführung des erfindungsgemäßen Reinigungs- bzw. Isolierungsverfahrens oder Probenvorbereitungsverfahrens enthält.

Die vorliegende Erfindung weist den Vorteil auf, daß die in Stufe e) des Verfahrens erhaltenen (hoch)gereinigten Nukleinsäuren nicht mit PEG kontaminiert sind, da dieses durch das Waschen der an der Matrix gebundenen Nukleinsäuren vollständig entfernt wird. Sie sind ferner derart angereichert, daß mit Hilfe kommerziell erhältlicher NAT-Assays noch etwa 6 IU/ml HCV-RNA (entsprechend ca. 20 RNA-Kopien pro ml) nachgewiesen werden können. Für HBV und HIV-1 wird eine entsprechende Nachweisgrenze erreicht.

Diese Nachweisgrenze ermöglicht insbesondere ein Testen von Proben aus Plasma-Pools, wobei die vom Paul-Ehrlich-Institut gesetzten Anforderungen im Hinblick auf eine Mindest-Empfindlichkeit von 5000 IU HCV/ml pro Einzelprobe nicht nur erfüllt sondern um einiges (um einen Faktor von mind. etwa 8 in 96er-Pools) übertroffen werden. Durch die vorliegende Erfindung ist es beispielsweise erstmals möglich, Plasma-Pools zu testen, bei denen nur eine von 96 Einzelproben mit 1000 IU/ml HCV kontaminiert ist, was einer Konzentration von etwa 10 IU/ml im Pool entspricht.

Plasma-Pools lassen sich somit erstmals mit kommerziellen NAT-Tests mit hoher Sensitivität auf das Vorhandensein von Viruspartikeln testen, wobei aufgrund der durch die Probenvorbereitung stark verbesserten Sensitivität bzw. deutlich herabgesetzten Nachweisgrenze das Erfordernis zur Einzelproben-Untersuchung entfällt.

Die Erfindung findet vorzugsweise Anwendung zum Nachweis von HCV, HBV und HIV-1 aus gepoolten Plasmen von Blutspendediensten, die derzeit als Standard 96 Einzelproben umfassen.

Das erfindungsgemäße Reinigungs- bzw. Isolierungsverfahren zeichnet sich gegenüber einem Ultrazentrifugations-Protokoll zur Anreicherung von Viruspartikeln aus Flüssigkeiten dadurch aus, daß auf teure Ultrazentrifugen-Ausstattung verzichtet werden kann, da jede Standard-Kühl-Zentrifuge, die 5 - 15 ml Röhrchen aufnehmen kann, zur Durchführung des Verfahrens geeignet ist. Dadurch ist im Rahmen der vorliegenden Erfindung insbesondere auch eine parallele Bearbeitung einer großen Anzahl von Proben möglich, d.h. in der Regel mehr als 60 Proben, während bei Verwendung von Ultrazentrifugen infolge der meist sehr geringen Anzahl vorhandener Plätze für Röhrchen in der Zentrifuge (oft weniger als 12) nur wenige Proben gleichzeitig bearbeitet werden können.

Durch das Verfahren der vorliegenden Erfindung wird im Vergleich zur Ultrazentrifugation ferner eine schnellere Sedimentierung der Viruspartikel (ca. 40 min genüber mehreren Stunden) erreicht.

Bei erfindungsgemäß durchgeführter Probenvorbereitung erfordert das gesamte Verfahren zum Nachweis von Viren in Flüssigproben unter Einsatz kommerziell erhältlicher NAT-Assays somit nicht mehr als etwa 7 Stunden. Dadurch kann es auch dann mit Erfolg eingesetzt werden, wenn von der Testung die Freigabe nur für kurze Zeit (3-4 Tage) haltbarer Blutprodukte, wie z.B. Thrombozyten, abhängt. Im Gegensatz dazu benötigt man derzeit für Nachweisverfahren, bei denen die Anreicherung der Viruspartikel ausschließlich durch Ultrazentrifugation erreicht wird, etwa 2 Tage, welches für die Freigabe von Thrombozyten nicht anwendbar ist. Durch die vorliegende Erfindung wird somit erstmals die hochsensitive Testung von Blutprodukten (wie z.B. von Thrombozyten) gestattet, die nur für relativ kurze Zeit haltbar sind.

Das erfindungsgemäße Verfahren zeichnet sich ferner dadurch aus, daß insgesamt weniger Probenmaterial zur Durchführug der NAT-Assays eingesetzt werden muß, da anstelle der Einzelproben der gesamte Plasma-Pool getestet werden kann. Ferner ist das Verfahren aufgrund des geringeren Kostenaufwands sowie der enormen Zeitersparnis für Blutspendedienste und Blutbanken, wo mehrere 1000 Proben pro Tag getestet werden müssen, besonders geeignet.

Die Erfindung weist außerdem den besonderen Vorteil auf, daß auch lipid-assoziierte Viruspartikel (z.B. HCV) quantitativ präparierbar und mit hoher Sensitivität nachweisbar sind, während sie bei Anreicherung durch Ultrazentrifugation in der oben schwimmenden Fettphase verbleiben können.

Gegenüber Standard-Protokollen zur Isolierung von Virus-Nukleinsäuren aus Flüssigkeiten besitzt das erfindungsgemäße Verfahren den Vorteil, daß man nicht auf nur sehr geringe Test-Volumina (100-200 µl) festgelegt ist, da Proben von bis zu 10 ml Volumen eingesetzt werden können. Durch die infolge der erfindungsgemäßen Probenvorbereitung gegenüber dem Stand der Technik um einen Faktor 8 bis 100 erhöhte Empfindlichkeit von 20 oder weniger nachweisbaren Viruskopien/ml ist es möglich, Viren auch in Proben mit sehr geringem Virustiter nachzuweisen bzw. zu quantifizieren. Auf diese Weise wird eine erhöhte Sicherheit bei der Verwendung von Blut(plasma)produkten erzielt.

Die vorliegende Erfindung wird nachfolgend anhand von Beispielen näher erläutert.

### Beispiel 1

### Allgemeines Protokoll zur Isolierung/Reinigung viraler Nukleinsäuren

### Fällung:

Aus einem großen (Plasma-) Probenvolumen (max. 10 ml) werden Viruspartikel konzentriert. Dies geschieht duch Fällung mit PEG 6000 (ca. 3% Gewichts-Volumenanteil, d.h. 50 g PEG ad 100 ml H₂O (w/v)) und NaCl (ca. 150 mM einer wässrigen Lösung) für ca. 15-20 min in der Kälte (4°C) mit anschließender Zentrifugation (15-20 min, 4°C, 3000 g).

### Extraktion:

Nach Dekantieren des Überstandes wird das Pellet in 300-400 µl GIT-Puffer (6 M GIT, 200 mM DTT, 100 mM Tris, pH 6,5) durch Inkubation bei ca. 56°C für ca. 10 min gelöst.

### Reinigung der Nukleinsäuren:

Zur Vorbereitung der Bindung der Nukleinsäuren an die Silicia-Membran wird das Lysat mit dem Lysis-Puffer (s.o.) eines kommerziellen Virus RNA Isolierungskits (z.B. QIAamp Viral RNA Kit, QIAGEN, oder High Pure Viral RNA Kit, Boehringer) im Verhältnis 1:1 gemischt und anschließend mit dem doppelten Volumen EtOH (96-99 %) versetzt.

Das Nukleinsäure-GIT-EtOH Gemisch wird im nächsten Schritt auf eine Nukleinsäure-bindende Silicia-Membran gegeben (als Zentrifugations-Säulchen in der manuellen oder als Multiwell 96er-System in der maschinellen Variante verschiedener kommerzieller Anbieter, s.o.) und nach Testvorschrift des Herstellers bis zur Elution der gereinigten RNA/DNA weiter verarbeitet.

### Beispiel 2

### Spezielles Anwendungsprotokoll - Nachweis von HCV in Blutplasma

4,8 ml eines 96er-Plasmapools werden in einem 7 ml Zentrifugationsröhrchen mit 300 µl 50 % PEG 6000 und 150 µl 5 M wässrigem NaCl versetzt, gemischt und für ca. 15-20 min bei 4°C inkubiert.

Anschließend werden die Röhrchen für 15-20 min bei 4°C mit 3000 g zentrifugiert, um die ausgefällten PEG/Virus-Komplexe zu sedimentieren.

Nach Dekantieren des Überstandes werden je nach Protokoll der Nukleinsäure-Reinigung (manuell oder maschinell, s.u.) 300-400 µl GIT-Puffer zum Pellet gegeben und dieses durch Inkubation bei ca. 56°C für ca. 10 min gelöst.

Das Lysat wird mit 300-400 µl Lysis-Puffer eines kommerziellen Virus RNA Isolierungskits (z.B. QIAamp Viral RNA Kit (s.u.), QIAGEN, oder High Pure Viral RNA Kit, Boehringer) im Verhältnis 1:1 gemischt und anschließend mit 600-800 µl 96-99 %igem EtOH versetzt.

Das Gemisch wird z.B. bei der manuellen Variante unter Verwendung des QIAamp Viral RNA Kits in zwei 630 µl Portionen auf die Zentrifugationssäulchen (Bestandteil des "Viral RNA Kits", s.o.) gegeben und nach Herstellervorschrift weiter vorgegangen. Bei der maschinellen Variante (QIAamp 96 Viral RNA Test Kit) werden die 7 ml Röhrchen mit dem Lysis-Gemisch in die Primärröhrchen-Adapter des BioRobot 9604 (QIAGEN) gestellt und entsprechend automatisch bis zur Elution der Nukleinsäuren prozessiert.

Zur nachfolgenden Detektion von Virusnukleinsäuren kann jedes etablierte NAT-Verfahren (z.B. PCR) Anwendung finden, das den Einsatz von 10-50 µl in H₂O (oder in einem vom Kit-Hersteller gelieferten Elutionspuffer) eluierter Nukleinsäure gestattet.

### Beispiel 3

Das in Beispiel 2 dargestellte Verfahren wurde anhand von mehreren hundert von ihrer Virusmenge her definierten Proben getestet und eine Nachweisgrenze von ca. 20 Viruskopien/ml durch Einsatz der Nukleinsäure-Eluate in die Amplicor Testsysteme HBV Monitor, HCV Test und HIV-1 Monitor (Hoffmann-La Roche) ermittelt. Die Ergebnisse sind in Tab. 1 dargestellt.

**Tab. 1:**

| minimale Nachweisgrenzen (Mittelwerte in Nukleinsäurekopien/ml) der Amplicor HCV® v2.0, HBV Monitors und HIV-1 Monitor® v 1.5 Tests gemäß Standardprotokollen im Vergleich zur erfindungsgemäßen Durchführung des Verfahrens | | | | |
|---|---|---|---|---|
| Virus | Erfindung* | HBV Monitor | HCV v.2.0 | HIV-1 Monitor |
| HBV | < 20 DNA/ml | ca. 400 DNA/ml | - | - |
| HCV | < 20 RNA/ml | - | ca. 100 RNA/ml | - |
| HIV-1 | < 20 RNA/ml | - | - | ca. 400 RNA/ml |

| | | | | |
|---|---|---|---|---|
| *bezogen auf die Probenvorbereitung (Nukleinsäure-Extraktion) gemäß Beispiel 2; zum Amplifizieren wurden die Reagenzien der Amplicor-Tests verwendet. | | | | |

## Patentansprüche

1. Verfahren zur Reinigung und Isolierung von Nukleinsäuren von in biologischem Probenmaterial vorhanden Viruspartikeln, **dadurch gekennzeichnet, dass** man
a) eine Virus-enthaltende Probe mit Polyethylenglycol und Natriumchlorid versetzt und bei etwa 4°C Viruspartikel ausfällt,
b) eine Zentrifugation durchführt,
c) erhaltene Pellets in Guanidinisothiocyanat-Puffer löst,
d) zu der in c) erhaltenen Mischung Ethanol zugibt, und
e) eine Reinigung an einer Silicia-Matrix durchführt,
wobei die gereinigten Nukleinsäuren anschliessend im Eluat vorliegen.

2. Verfahren nach Anspruch 1, **dadurch gekennzeichnet, daß** man in Stufe d) die in c) erhaltene Mischung zunächst mit dem gleichen Volumen Lysis-Puffer oder Guanidinisothiocyanat-Puffer mischt und anschliessend das doppelte Volumen Ethanol zugibt.

3. Verfahren nach Anspruch 1 oder 2, **dadurch gekennzeichnet, dass** das biologische Probenmaterial aus der Gruppe bestehend aus Plasma, anderen Blutprodukten sowie anderen Flüssigkeiten ausgewählt ist.

4. Verfahren nach Anspruch 2 oder 3, **dadurch gekennzeichnet, dass** man
in Stufe a) eine Plasmaprobe verwendet und zu einem Probenvolumen von max. ca. 10 ml 3 Vol.% Polyethylenglycol 6000 eine wässrige Natriumchloridlösung bis zu einer Endkonzentration von ca. 150 mM zugibt und man für ca. 15-20 Min. bei 4°C stehen läßt,
in Stufe b) für 15-20 Min. bei 4°C und ca. 3000 g zentrifugiert,
in Stufe c) zum Lösen des Pellets 300 bis 400 µl Guanidinisothiocyanat-Puffer zugibt und für ca. 10 Min. bei 56°C löst und in Stufe d) die Lösung mit Lysis-Puffer im Verhältnis 1:1 mischt.

5. Verfahren nach einem der Ansprüche 2 bis 4, **dadurch gekennzeichnet, dass** man
a) 4,8 ml eines 96er Plasmapools in einem 7 ml Zentrifugationsröhrchen mit 300 µl 50%igem Polyethylenglykol 6000 und 150 µl einer 5M-wässrigen Natriumchloridlösung ersetzt, mischt und für ca. 15-20 Minuten bei 4°C inkubiert,
b) für 15-20 Min. bei 4°C mit 3000 g zentrifugiert,
c) 300 bis 400 µl Guanidinisothiocyanat-Puffer zu dem Pellet gibt und man dieses durch Inkubation bei ca. 56°C für ca. 10 Min. löst,
d) das Lysat mit 300 bis 400 µl Guanidinisothiocyanathaltigem Lysispuffer im Verhältnis 1:1 mischt und anschliessend mit 600 bis 800 µl Ethanol versetzt und
e) eine Reinigung an Silicia-Matrix durchführt,
wobei die gereinigten Nukleinsäuren im Eluat vorliegen.

6. Verfahren zum Nachweis von Viren aus gepoolten Plasmen von Blutspendern, **dadurch gekennzeichnet, dass** man
a) Nukleinsäuren von in biologischem Probenmaterial vorhandenen Viruspartikeln nach dem Verfahren gemäß der Ansprüche 1-5 reinigt und isoliert, und
b) anschließend die Virus-RNA mit Hilfe einer Nukleinsäureamplifikationstechnik amplifiziert, und
c) die amplifizierte Virus-RNA nachweist.

7. Verfahren nach Anspruch 6, **dadurch gekennzeichnet, daß** die Viren HCV, HBV und/oder HIV sind.

8. Verfahren nach Anspruch 6und 7, **dadurch gekennzeichnet, dass** die Nukleinsäure-Amplifikationstechnik PCR, bDNA oder NASBA ist.

9. Kit zur Durchführung des Verfahrens nach einem der Ansprüche 1 bis 5, **dadurch gekennzeichnet, dass** er Polyethylenglykol. Natriumchlorid, Guanidinisothiocyanat-Puffer Ethanol und eine Silicia-Matrix enthält.

10. Kit nach Anspruch 9, **dadurch gekennzeichnet, dass** das Polyethylenglykol Polyethylenglykol 6000 ist.

11. Kit nach Anspruch 10, **dadurch gekennzeichnet, dass** es zur Vorbereitung einer Plasmaprobe von je 4,8 ml Volumen
a) 300 µl 50%iges Polyethylenglykol 6000,
b) 150 µl einer 5 M wässrige Natriumchlorid-Lösung,
c) 400 µl Guanidinisothiocyanat-Puffer und
d) 800 µl Ethanol
enthält.

12. Kit zur Durchführung des Verfahrens nach Anspruch 6 und 7, **dadurch gekennzeichnet, dass** es die Bestandteile eines Kits nach den Ansprüchen 9 bis 11 sowie zur Durchführung einer Nukleinsäure-Amplifikation und zum Nachweis der amplifizierten Nukleinsäure erforderliche Bestandteile enthält.

13. Kit nach Anspruch 12, **dadurch gekennzeichnet, dass** die Nukleinsäure-Amplifikation PCR, bDNA oder NASBA ist.

## Claims

1. Method for purifying and isolating nucleic acids of virus particles present in a biological sample material, **characterized in that**
a) polyethylene glycol and sodium chloride are added to a virus-containing sample and virus particles are precipitated at approximately 4°C,
b) a centrifugation is carried out,
c) pellets obtained are dissolved in guanidine isothiocyanate buffer,
d) ethanol is added to the mixture obtained in c), and
e) a purification is carried out on a silica matrix, and the purified nucleic acids can subsequently be found in the eluate.

2. Method according to Claim 1, **characterized in that** in stage d) the mixture obtained in c) is first mixed with the same volume of lysis buffer or guanidine isothiocyanate buffer and then twice the volume of ethanol is added.

3. Method according to Claim 1 or 2, **characterized in that** the biological sample material is selected from the group consisting of plasma, other blood products and other fluids.

4. Method according to Claim 2 or 3, **characterized in that**
in stage a) a plasma sample is used and an aqueous sodium chloride solution is added to a final concentration of approx. 150 mM to a sample volume of no more than approx. 10 ml of 3% by volume polyethylene glycol 6000 and the mixture is left at 4°C for approx. 15-20 min,
in stage b) a centrifugation is carried out at 4°C and approx. 3 000 g for 15-20 min,
in stage c) 300 to 400 µl of guanidine isothiocyanate buffer are added to dissolve the pellet subsequently at 56°C for approx. 10 min and in stage d) the solution is mixed with lysis buffer in a 1:1 ratio.

5. Method according to any of Claims 2 to 4, **characterized in that**
a) 4.8 ml of a 96 plasma pool in a 7-ml centrifuge tube are replaced with 300 µl of 50% strength polyethylene glycol 6000 and 150 µl of an aqueous 5M sodium chloride solution, mixed and incubated at 4°C for approx. 15-20 minutes,
b) a centrifugation is carried out at 4°C and 3 000 g for 15-20 min,
c) 300 to 400 µl of guanidine isothiocyanate buffer are added to the pellet which is then dissolved by incubation at approx. 56°C for approx. 10 min,
d) the lysate is mixed in a 1:1 ratio with 300 to 400 µl of guanidine isothiocyanate-containing lysis buffer, followed by the addition of 600 to 800 µl of ethanol, and
e) a purification on a silica matrix is carried out,
and the purified nucleic acids can be found in the eluate.

6. Method for detecting viruses from pooled plasmas of blood donors, **characterized in that**
a) nucleic acids of virus particles present in biological sample material are purified and isolated by the method according to Claims 1-5, and
b) the viral RNA is subsequently amplified with the aid of a nucleic acid amplification technique, and
c) the amplified viral RNA is detected.

7. Method according to Claim 6, **characterized in that** the viruses are HCV, HBV and/or HIV.

8. Method according to Claim 6 and 7, **characterized in that** the nucleic acid amplification technique is PCR, bDNA or NASBA.

9. Kit for carrying out the method according to any of Claims 1 to 5, **characterized in that** it contains polyethylene glycol, sodium chloride, guanidine isothiocyanate buffer, ethanol and a silica matrix.

10. Kit according to Claim 9, **characterized in that** the polyethylene glycol is polyethylene glycol 6000.

11. Kit according to Claim 10, **characterized in that**, in order to prepare a plasma sample of in each case 4.8 ml in volume, it contains
a) 300 µl of 50% strength polyethylene glycol 6000,
b) 150 µl of an aqueous 5M sodium chloride solution,
c) 400 µl of guanidine isothiocyanate buffer, and
d) 800 µl of ethanol.

12. Kit for carrying out the method according to Claim 6 and 7, **characterized in that** it contains the components of a kit according to Claims 9 to 11 and the components required for carrying out a nucleic acid amplification and for detecting the amplified nucleic acid.

13. Kit according to Claim 12, **characterized in that** the nucleic acid amplification is PCR, bDNA or NASBA.

## Revendications

1. Procédé de purification et d'isolement d'acides nucléiques de particules virales présentes dans des échantillons biologiques, **caractérisé en ce que**
a) on ajoute à l'échantillon contenant le virus, du polyéthylèneglycol et du chlorure de sodium et on précipite les particules virales à environ 4°C ;
b) on réalise une centrifugation;
c) on dissout les culots obtenus dans du tampon isothiocyanate de guanidine ;
d) on ajoute de l'éthanol au mélange obtenu en c), et
e) on réalise une purification sur une matrice de silice, où les acides nucléiques purifiés étant présents ensuite dans l'éluat.

2. Procédé selon la revendication 1, **caractérisé en ce que** l'on mélange à l'étape d), le mélange obtenu en c), d'abord avec le même volume de tampon de lyse ou de tampon isothiocyanate de guanidine et ensuite, avec deux fois le volume d'éthanol.

3. Procédé selon la revendication 1 ou 2, **caractérisé en ce que** l'échantillon biologique est choisi parmi le groupe consistant en du plasma, d'autres produits sanguins ainsi que d'autres liquides.

4. Procédé selon la revendication 2 ou 3, **caractérisé en ce que**
on utilise à l'étape a), un échantillon de plasma et on ajoute à un volume de l'échantillon de maximum environ 10 ml de polyéthylèneglycol 6000 à 3% en volume, une solution aqueuse de chlorure de sodium jusqu'à une concentration finale d'environ 150 mM et on laisse à 4°C pendant environ 15-20 minutes ;
on centrifuge à l'étape b), pendant 15-20 minutes à 4°C et environ 3000 g ;
on ajoute à l'étape c), pour dissoudre le culot, 300 à 400 µl de tampon isothiocyanate de guanidine et on dissout pendant environ 10 minutes à 56°C et on mélange à l'étape d), la solution avec le tampon de lyse en un rapport de 1:1.

5. Procédé selon l'une quelconque des revendications 2 à 4, **caractérisé en ce que**
a) on ajoute à 4,8 ml d'un ensemble de 96 plasmas dans un tube à centrifuger de 7 ml, 300 µl de polyéthylèneglycol 6000 à 50% et 150 µl d'une solution aqueuse de chlorure de sodium 5 M, on mélange et on incube à 4°C pendant environ 15-20 minutes ;
b) on centrifuge pendant 15-20 minutes à 4°C à 3000 g ;
c) on ajoute au culot, 300 à 400 µl de tampon isothiocyanate de guanidine et on dissout celui-ci par incubation à environ 56°C pendant environ 10 minutes ;
d) on mélange le lysat avec 300 à 400 µl de tampon de lyse contenant l'isothiocyanate de guanidine en un rapport 1 :1 et ensuite, on ajoute 600 à 800 µl d'éthanol, et
e) on réalise une purification sur matrice de silice,
les acides nucléiques purifiés se trouvant dans l'éluat.

6. Procédé de détection de virus dans des plasmas rassemblés de donneurs de sang, **caractérisé en ce que**
a) on purifie et isole les acides nucléiques des particules virales présentes dans l'échantillon biologique, d'après le procédé selon les revendications 1-5, et
b) on amplifie l'ARN viral à l'aide d'une technique d'amplification d'acide nucléique, et
c) on détecte l'ARN viral amplifié.

7. Procédé selon la revendication 6, **caractérisé en ce que** les virus sont HCV, HBV et/ou HIV.

8. Procédé selon les revendications 6 et 7, **caractérisé en ce que** la technique d'amplification d'acide nucléique est la PCR, la bDNA ou la NASBA.

9. Kit pour réaliser le procédé selon les revendications 1-5, **caractérisé en ce qu'**il contient du polyéthylèneglycol, du chlorure de sodium, le tampon isothiocyanate de guanidine, de l'éthanol et une matrice de silice.

10. Kit selon la revendication 9, **caractérisé en ce que** le polyéthylèneglycol est le polyéthylèneglycol 6000.

11. Kit selon la revendication 10, **caractérisé en ce qu'**il contient pour le traitement d'un échantillon de plasma d'un volume de chaque fois 4,8 ml
a) 300 µl de polyéthylèneglycol 6000 à 50% ;
b) 150 µl d'une solution aqueuse de chlorure de sodium 5 M ;
c) 400 µl de tampon isothiocyanate de guanidine, et
d) 800 µl d'éthanol.

12. Kit pour réaliser le procédé selon les revendications 6 et 7, **caractérisé en ce qu'**il contient les constituants d'un kit selon les revendications 9 à 11, ainsi que les constituants nécessaires à la réalisation d'une amplification d'acide nucléique et à la détection des acides nucléiques amplifiés.

13. Kit selon la revendication 12, **caractérisé en ce que** l'amplification d'acide nucléique est la PCR, la bDNA ou la NASBA.
